# EUROPEAN PATENT APPLICATION

(11) **EP 0 820 706 A2**
(43) Date of publication of application: **28.01.1998**
(21) Application number: 97202791.6
(22) Date of filing: 09.11.1990
(51) Int. Cl.: A43B 7/28, A43B 7/22, A61F 5/14

(54) **Improvements in or relating to orthotic devices**

(30) Priority: 17.11.1989 AU PJ7446/89
(62) Divisional of application: 90916436.0
(71) Applicant: PARAMOUNT CAPITAL EXCHANGE CORPORATION LTD., Hamilton, HM11 (BM)
(72) Inventor: Vasyli, Philip John, Nassau, N.P. (BS)
(74) Representative: Findlay, Alice Rosemary

(57) **Abstract**

A contoured, mouldable orthotic device (1) of about three-quarters the length of the boot or shoe in which it is to fit, wherein the device has an integrally-formed heel cup (2), a longitudinal arch "raise" (3), a metatarsal "raise" (A) and a varus post (θ). The device is placed in the boot or shoe as far back as it will go and then heated. The patient's foot is introduced into the boot or shoe and the now-pliable orthotic device is moulded and otherwise manipulated to the foot.

## Description

This invention relates to orthotic devices and more particularly to such a device which is able to be moulded to a patient's foot, "in situ" in an article of footwear, to give support to, and to control, the osseous structures of the foot.

Foot support devices are known, ranging from simple contoured insoles to costly structures integrally incorporated into made-to-order orthopaedic footwear.

Orthopaedic footwear apart, known insertable insole devices as a general rule overlie the whole of the upper surface of the line of the shoe sole, thus requiring the existence of an extensive range of lengths, widths and even shapes - for example, the court-fit shape in women's dress shoes. These known insoles are usually sold "off the shelf" and provide if any, but indifferent biomechanical control or if they are fitted to the patient's shoe, the fitting and adjustment time may be quite long. They rarely provide satisfactory bio-mechanical control for, and control of, the osseous structures of the foot and are often made of unyielding materials such as hard plastic or carbon fibre; on the other hand, many resilient moulded insoles merely function as shock absorbers.

"Full length" devices are disclosed in such U.S. Patents as No. 3,782,390 (JOHNSON) in which is taught a method of producing inserts by injecting a curable fluid into a shoe 12 in which is a foot 8; No. 2,760,281 (COSIN) discloses an insole or insert composed of several discrete layers including a "sandwiched" triangular element 15; and No. 2,409,594 (SHERMAN) discloses a corrective foot appliance formed from sheet material which is "capable of becoming relatively permanently set in shape in the course of time when treated with an activator.".

US Patent 3,895,405 (EDWARDS) discloses a full length device which cushions the foot and whose function is simply comfort-related as a shock absorber. The device may be moulded following heating in situ to the patient's foot from an initially flat sheet to conform to the contours of the foot.

U.S. Patent Nos. 4,674,204; 4,232,457; 3,825,017 and 4,522,777 to SULLIVAN, MOSHER, SCRIMA and PETERSON respectively disclose various orthotic insoles having spongy or foam layers.

US Patent 4,232,457 is included in the above list as showing an orthotic insert, but the device disclosed is primarily an arch support used to prevent excessive pronation of the foot at both heel strike and mid stance and points between which causes the tibia and fibula to rotate inwardly placing strain on the leg muscles on the inner side of the knee and in the plantar fascia.

Generally, a "corrective" orthotic is a device which has a structure and surfaces such that the alignment and position of the mechanical structure of the wearer's foot are adjusted and corrected so as to improve the gait, posture and other biomechanical aspects of the wearer patient's foot are improved or corrected. The device maintains the natural intrinsic control of the osseous (bony) structures of the foot, allowing the foot to function about its neutral sub-talar and mid-tarsal joint positions.

An arch support is a device having a structure and surfaces that contact the wearer's foot such that the foot is supported without control of movement around the neutral sub-talar and mid-tarsal joint positions. A cushioning arch-support flattens and forms to the wearer's foot under pressure providing cushioning comfort but no correction of the osseous structures.

An arch support is disclosed in US Patent 2,401,514 (SCHOLL) where the inventive concept is a strip of fabric 15 applied to the underside of a thermoplastic resin arch support to prevent it from sliding and squeaking when worn inside a shoe.

U.S. Patent No. 4,517,981 (SANTOPIETRO) relates to a substantially flat, three-quarter length orthotic device having no longitudinal arch "raise" or metatarsal "raise". U.S. Patent No. 4,823,420 (BARTNECK) discloses a contour moulded insole, including several layers of material; it is apparently somewhat less than three-quarter length and it is arbitrarily cut off in a straight transverse front edge provided with no metatarsal "raise".

Other specifications of interest are U.S. Patent Nos 2,653,396 (GOTTLIEB); 3,068,872 (BRODY); 3,121,431 (ROSENHAFT) ; 3,859,740 (KEMP); 3,309,797 (POATIS); 4,216,778 (WEISS); 4,268,980 (GUDAS); 4,346,525 (LARSEN) ; 4,364,188 (TURNER); 4,463,761 (POIS); 4,520,581 (IRWIN); 4,530,173 (GESINSKY) ; 4,557,060 (KAWASHIMA) ; 4,563,787 (DREW) ; 4,674,201 (WEISS) : 4,686,993 (GRUMBINE) ; 4,702,255 (SCHENKI); 4,756,095 (MAYER); 4,791,736 (PHILLIPS) and 4,868,945 (DE VETTIGNIS).

British Patent 1066996 describes an arch support device which moulds to the surface of a wearer's foot under the pressure of the wearer's weight, the material and shape being such that the device is restored to its original shape on removal of body pressure. The device as supplied may include an elevation to give support to the metatarsal or transverse arch of the foot and a heel seat. It is, however, completely reshapable under heating by placement in a pan of hot water. The device functions primarily, due to its deformable nature, as a cushioning arch support rather than a corrective orthotic.

EP-A-0154170 discloses an arrangement for moulding an insole from an essentially formless pad. The moulded features are essentially simply a cast of the patient's foot.

It is an object of the present invention to overcome or, at least, mitigate certain disadvantages and shortcomings of the prior art.

In accordance with the invention a contoured mouldable orthotic device, said device being operatively positioned between an article of footwear and a user's foot so as to overlie at least part of the upper surface of the sole of the article of footwear and operatively underlie the heel bone and head ends of the metatarsals of the user's foot, wherein the device is formed with orthotic structures on its upper contour, said orthotic structures including an integrally moulded heel cup, a longitudinal arch raise, a metatarsal raise for aligning the heads of the second, third and fourth metatarsal bones, and a varus post of about 4 degrees, the device being of a length so as to operatively position the heel cup to underlie the heel bone and the metatarsal rise to underlie the head ends of the second, third and fourth metatarsal bones of said user's foot, and wherein the device is formed from a material such that the upper contour of the orthotic device is adapted to be altered by heating the orthotic device immediately followed by imposing pressure from the user's foot so as to customise the upper contour while simultaneously retaining said orthotic structures.

There is thus provided a contoured mouldable orthotic device having certain pre-determined shapes and angles and adapted to be inserted into an article of footwear so as to overlie part of the upper surface of a sole thereof and to be able to subsequently moulded in situ to a patient's foot to thereby give support to, and to control, the osseous structure thereof. The length of said mouldable orthotic device is such as to underlie the heel bone and to terminate adjacent the head ends of the metatarsal of a said patient's foot; said orthotic device being formed with an integrally moulded heel cup, a longitudinal arch raise, a varus post of about 4°, and a metatarsal raise for aligning the heads of the second, third and fourth metatarsals.

Ideally, the width of the mouldable orthotic device is that distance from the lateral aspect of the head of the fifth metatarsal to the longitudinal bisection of the first and second metatarsals; the arrangement being such that the shaft of the first metatarsal is able to plantarflex during the propulsive phase of a said patient's foot. The length is substantially three-quarters of the length of the article of footwear.

The terms "raise" and "plantarflex" are well-understood by those familiar with the field of orthotic devices.

In order that a better understanding of the present invention may be gained, hereinafter will be described preferred embodiments thereof, by way of example only and with reference to the accompanying drawings in which:-
Figure 1 is a side elevation of the right-hand side of a left-foot mouldable orthotic device;
Figure 2 is a bottom plan view;
Figure 3 is a side elevation of the left-hand side of the device;
Figure 4 is a top plan view;

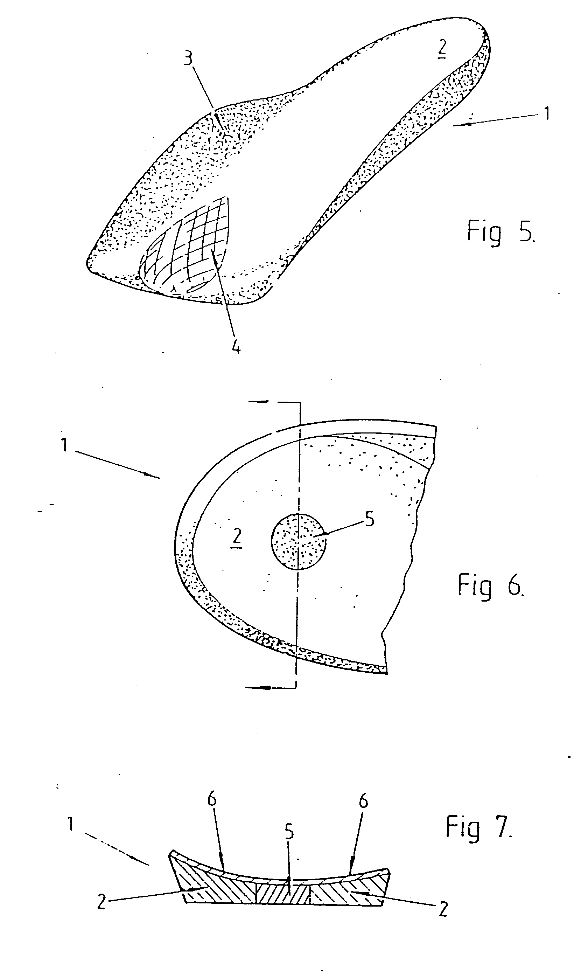

Figure 5 is a frontal top perspective;
Figure 6 is a fragmentary view of the bottom of the heel portion of another embodiment which is provided with a shock-absorbing, spongy "shock dot" insert; and
Figure 7 is a cross-section along line VII-VII of Figure 6, but somewhat exaggerated as regards proportions.

Throughout the drawings, like integers are referenced by the same numeral and, throughout the specification, the adjective "orthotic" is used to qualify "device", instead of the alternatives "orthotic", "orthopedic" or "orthopaedic".

In the drawings there is shown a mouldable orthotic device, generally referenced 1, which may well be of such a material as 350 Kg/M³ density "ETHYL VINYL ACETATE" (E.V.A.) or 220 Kg/M³ density "ETHYL VINYL ACETATE" (E.V.A.). The specifications of these two materials are as in the following table:-

| PROPERTY & TEST METHOD | UNITS | "E.V.A." | "E.V.A." |
|---|---|---|---|
| DENSITY | Kg/M³ | 350 | 220 |
| HARDNESS (jis type C) | - | 74 | 57 |
| WATER ABSORPTION (jis K6767) | gms/cm³ | <0.002 | <0.002 |
| THERMAL CONDUCTIVITY (astm 578) | W/M°C. | 0.064 | 0.055 |
| OPERATING TEMPERATURE | Min °C | -70°C | -70°C |
| | Max °C | 70°C | 70°C |
| TENSILE STRENGTH (jis K6767) | MPa | 3.0 | 2.0 |
| TEAR STRENGTH (jis K6767) | N/Cm | 170 | 120 |
| ELONGATION AT BREAK (jis K6767) | % | 250-300 | 250-300 |
| COMPRESSION SET | % | < 5 | < 5 |
| COMPRESSION DEFLECTION (astm d3575) | KPa | 950 | 240 |
| | | | |

The mouldable orthotic device may be provided in various geometries, that is to say, in various shapes, as regular adult; women's "court fit"; infants; and youths. Children's size ranges envisaged are, say, 1 - 3; 4 - 6; 7 - 9; 10 - 12; and 13 youth's size 2; in regular and wide fittings: thus, ten 'models' are provided. Also envisaged are adult's sizes 4 - 6; 7 - 9; and 10 - 12; in regular, wide and "court" fittings, nine adult models being provided in this range.

Each mouldable orthotic device 1 is formed of E.V.A. from a positive cast based on that same configuration as are known rigid devices. The length of the device is substantially three-quarters of the length of the inside of the article of footwear so as to underlie the heel bone, (the calcaneus or os calcis,), terminating adjacent the heads of the metatarsals, which are those bones between the phalangeal bones and the seven tarsal bones that articulate the foot.

Each orthotic device 1 is formed with an integrally moulded heel cup 2, longitudinal arch "raise" 3, and a metatarsal raise 4 for the purpose of aligning the heads of the second, third and fourth metatarsals.

The width of the mouldable orthotic device ideally is from the lateral aspect of the fifth metatarsal bone's head to the longitudinal bisection of the first and second metatarsals. This arrangement permits the shaft of the first metatarsal bone to "plantarflex" during the propulsive phase - that is to say, during walking or running. If necessary, additional wedging and/or other geometric configurations might well be incorporated for the purpose of correcting severe fore-foot, midtarsal and/or rear-foot deformities.

It is contemplated that, initially at least, specifically-trained technical personnel will "custom-mould" the inventive mouldable orthotic devices onto existing pre-moulded cast templates; however, it is envisaged that along-term production process will combine in-house "cad/cam" manufacturing procedures with some utilisation of industrial moulding techniques.

For certain foot malformations, mouldable adjuncts may include 2°, 4°, 6°, or 8° angle adhesive wedges; 4 or 8 mm heel adhesive "raises" and fabric covers for the mouldable orthotic devices.

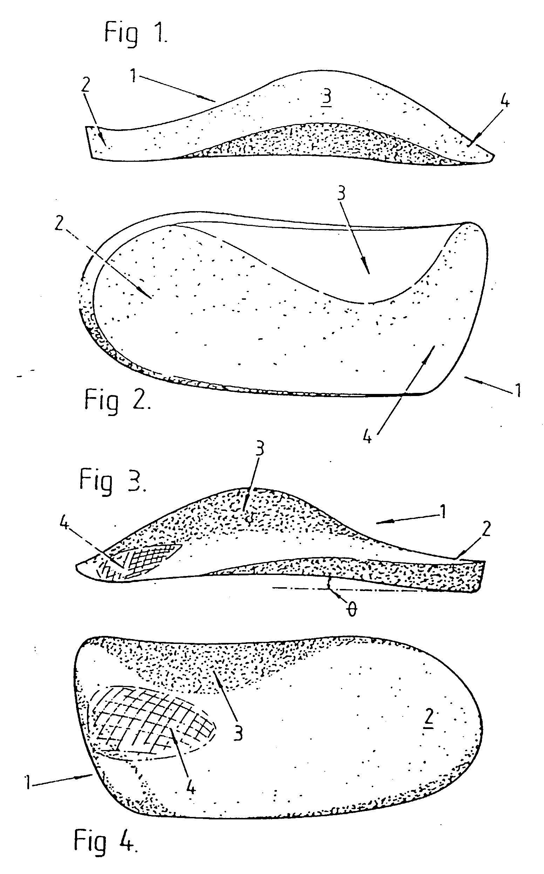

A 4° - angled (or thereabouts) rear foot post - a so-called "varus post" - as indicated by the angle θ shown in Figure 3, is in-built to allow for leg curvature and to prevent excess pronation.

In a modification, the pre-moulded orthotic device may incorporate a sponge-like, like shock-absorbing insert (highly preferably made of low-density polyurethane foam) 5 which is adapted to cushion that area immediately beneath the heel spur or calcaneus. Such an insert 5 may aptly be termed a "shock dot", or "shock spot". The cushioned area may extend either fully or partially through the full thickness of the orthotic device. Advantageously, the upper surface of the orthotic device may be covered, or sheathed, with a fabric-like outer "skin" - as referenced 6 in Figure 7.

A method for the "in situ" moulding to a patient's foot, of the mouldable orthotic device described above after the device has been inserted into an article of footwear so as to overlie part of the upper surface of its sole will now be described.

The moulding process is as follows:- A mouldable orthotic device as described above is inserted into a selected article of footwear, placed as far back as it will go. The device is then heated with a stream of hot air, from a suitable source, using a sequence cycle of about five seconds "on" followed by about five seconds "off". This cycle is repeated until such time as the device has undergone a total heating time of about twenty seconds. The patient is seated and his or her foot is fitted into the boot or shoe; palpate the subtalar joint in the known matter to neutral position. The patient is then required to stand, putting equal weight on each foot, while maintaining the neutral position of the subtalar joint. The medial and lateral edges of the article of footwear are pressed inwards so as to contour the warm mouldable orthotic device to the patient's neutral foot position. The device need only to be cooled for perhaps five minutes before the boot or shoe is ready for wearing, perhaps cooled to ambient temperature.

The procedure is then repeated for the other foot, if necessary; however, both articles of footwear of the pair should be worn to ensure equal balance during the moulding of each device. If required, fore-foot posts might well be applied to the plantar distal orthotic device's edge subsequent to the moulding process. Moreover, it will be realised than a mouldable orthotic device may be easily re-moulded if the desired result is not initially attained.

The present invention offers several distinct advantages over and above the prior art devices:- greater rear foot control action due to the high and solid heel cup; integrated cushioning "shock dot"; correctly contoured for metatarsal correction, longitudinal arch correction, lateral arch correction and sagittal calcaneal correction; greater stability due to the basic solidity of E.V.A. and to the wide surface rear foot area; in-built varus post, ideally 4°.

Tests have shown that the inventive mouldable orthotic device is well able to provide a remedy for common biomechanical problems relating to the foot as listed hereafter:- heel spurs; plantar fasciitis; Metatarsalgia; claw toes; calcaneal apophysitis; achilles tendonitis; shin splints; excess pronation; patella tracking malfunctions; flat feet; and like problems of an orthopaedic nature.

## Claims

1. A contoured mouldable orthotic device (1), said device being operatively positioned between an article of footwear and a user's foot so as to overlie at least part of the upper surface of the sole of the article of footwear and operatively underlie the heel bone and head ends of the metatarsals of the user's foot, wherein the device is formed with orthotic structures on its upper contour, said orthotic structures including an integrally moulded heel cup (2), a longitudinal arch raise (3), a metatarsal raise (4) for aligning the heads of the second, third and fourth metatarsal bones, and a varus post of about 4 degrees, the device being of a length so as to operatively position the heel cup to underlie the heel bone and the metatarsal rise to underlie the head ends of the second, third and fourth metatarsal bones of said user's foot, and wherein the device is formed from a material such that the upper contour of the orthotic device is adapted to be altered by heating the orthotic device immediately followed by imposing pressure from the user's foot so as to customise the upper contour while simultaneously retaining said orthotic structures.

2. A device as claimed in Claim 1, wherein the length of the device (1) is substantially three-quarters of the length of said article of footwear.

3. A device as claimed in Claim 2, wherein the device is tapered at the front edge so that operatively the device presents a smooth transition to the article of footwear.

4. A device as claimed in any preceding Claim, wherein the device has a lower surface which is substantially flat, but including a recess corresponding to the arch support on the upper surface.

5. A device as claimed in Claim 4, wherein the device is formed for a foam material and is essentially solid between the upper and lower surface.

6. A device as claimed in any preceding Claim, wherein the width of the device is the distance between the lateral aspect of the head of the fifth metatarsal and the longitudinal bisection of the first and second metatarsals, the arrangement being such that the shaft of the first metatarsal is able to plantar flex during the propulsive phase of a user's foot.

7. A device as claimed in any one of Claims 1 to 6, wherein the device (1) incorporates a shock-absorbing insert (5) adapted to cushion that area of a patient's foot which is immediately beneath the heel spur thereof.

8. A device as claimed in Claim 7, wherein said cushioned area extends either partially or fully through the full thickness of the device (1).

9. A device as claimed in any one of Claims 1 to 8, wherein the upper surface of said device (1) is sheathed in a fabric-like outer skin (6).

10. A device as claimed in any one of Claims 1 to 9, formed from ethyl vinyl acetate.

11. An article of footwear including the device (1) of any one of Claims 1 to 10.
